# EUROPEAN PATENT APPLICATION

(11) **EP 4 406 532 A1**
(43) Date of publication of application: **31.07.2024**
(21) Application number: 24154169.7
(22) Date of filing: 26.01.2024
(51) Int. Cl.: A61K 9/06, A61K 36/05, A61K 47/10, A61K 47/12, A61K 47/36, A61K 47/38, A61P 43/00, A61K 9/50

(54) **GEL FORMULATION FOR THE TREATMENT OF DRUG INTOXICATION AND A PROCESS TO OBTAIN THE SAME**

(30) Priority: 27.01.2023 PT 2023118487
(71) Applicant: Instituto Politécnico da Guarda, 6300-559 Guarda (PT)
(72) Inventor: Borges, Paula, 6300-538 Guarda (PT); Castro, Carla, 3500-187 Viseu (PT); Loureiro, Jorge, 3660-605 São Pedro do Sul (PT); Miguel, Sónia, 6200-160 Covilhã (PT); Jacinto, Telma, 6360-060 Celorico da Beira (PT); Moreira, André, 6200-506 Covilhã (PT); Filipe, Hugo, 2380-308 Espinheiro (PT); Silva, Luís, 3465-154 Santiago de Besteiros (PT); Ribeiro, Maximiano, 6200-815 Covilhã (PT); Saraiva, Sofia, 6300-090 6300-090 Guarda (PT)
(74) Representative: Couto, Cláudia

(57) **Abstract**

The present invention relates to a gel formulation for oral administration comprising microalgae biomass from *Chlorella sorokiniana, Chlorella vulgaris* or *Tetradesmus obliquus,* free or encapsulated in particles, suitable to adsorb different drugs in the gastrointestinal tract. It is also disclosed a process to obtain the gel formulation. The gel formulation is suitable for the treatment of drug intoxication in humans or animals, therefore finding application in pharmaceutical, medical and veterinary technical fields.

## Description

### Technical field

This application relates to a gel formulation for the treatment of drug intoxication and a process to obtain the same.

### Background art

Microalgae are photosynthetic organisms, eukaryotic and prokaryotic (cyanobacteria), and a diverse group adapted to survive in highly complex and competitive habitats, which exhibit a unique combination of typical features of higher plants combined with biotechnological attributes of microbial cells [1]. Due to these properties the microalgae biotechnological potential has been extensively studied in different fields such as bioremediation, cosmetics, biomedical and pharmaceutical industries, as well as in the development of functional foods and food supplements [2-5]. Additionally, several microalgae species are recognized as Generally Recognized as Safe (GRAS), which makes the use of microalgae as cell factories very appealing for pharmaceutical and food purposes [6]. Regarding the bioremediation applications, microalgae have been reported as promising efficient systems in the removal of various types of environmental pollutants, such as heavy metals and pharmaceuticals [7-10]. Such process occurs mainly through biosorption of contaminants (e.g. pharmaceuticals, heavy metals) by negatively charged cell wall of microalgae [9,11]. Such evidence was already stated for different microalgae species. Among the microalgae biodiversity, *Chlorella sorokiniana, Chlorella vulgaris , Tetradesmus obliquus* have been described as highly effective for drug removal in wastewater treatment, as described in different reports [12-15]. Also, the efficiency of *Phaeodactylum tricornutum* freeze-dried biomass was compared against fresh cultures in the removal of ibuprofen from seawater, demonstrating that there were no significant differences on the drug adsorption [16]. Furthermore, other authors evidenced the advantages of using freeze-dried microalgae biomass over living organisms, namely: i) possible storage at room temperature; ii) long durability without losing biosorptive properties; iii) absence of toxicity effects that may affect the treatment performance; iv) possibility of desorbing the pollutant and reutilization of microalgal biomass; and v) lower operational costs, including the need for growth media [9].

Despite of these attractive capacities of microalgae in drug removal from wastewater, these microorganisms were not yet explored in medical field. In fact, drug intoxications are considered a healthcare burden, given that only Portugal, there were 24,440 intoxications in 2021, of which 15,010 were caused by medicines. Regarding the class of drugs that most frequently are associated to intoxications, the data showed that the major culprits for poisonings were paracetamol (1202), alprazolam (1105), quetiapine (899), ibuprofen (803) and sertraline (703) [44].

Thus, the use of microalgal biomass seems to be a promising alternative for the conventional treatments (e.g. gastric lavage, hemodialysis, activated charcoal, N-acetylcysteine, flumazenil) used in drug intoxications [19,20], which present several limitations. For example, the efficacy of activated charcoal is highly dependent on time interval between ingestion and the treatment. Also, the gastric lavage carries serious risks, such as hypoxia, gastrointestinal perforations, and aspiration pneumonitis. On the other hand, the antidotes cannot be utilized as a universal method of treatment because they are specific to a limited number of toxins [21,22].

Apart from the human intoxication, another critical target is the animal intoxication, where it was estimated that vets treated about 323 dogs and 56 cats for poisoning every day across the UK. (DirectLine group, jan 2021). In Portugal, Centro de Informação Antivenenos (CIAV) reported that 353 dogs and 82 cats were intoxicated during 2021 (CIAV, 2021). So, it was equally relevant important sector that will benefits from such invention.

Given these, microalgae may also be an attractive approach to treat drug intoxication in humans, which constitute a major concern for healthcare system.

### Summary

The present invention relates to a gel formulation for the treatment of drug intoxication comprising:
- Between 0.5 - 5% (w/v) of microalga biomass selected from at least one of *Chlorella sorokiniana, Chlorella vulgaris* or *Tetradesmus obliquus;*
- Between 0.5% - 5% (w/v) of at least one polymer selected from alginate, carrageenan, pectin, chitosan, gum arabic, gellan gum, or glucomannan;
- Between 0.05% - 0.10% (w/v) of at least one preserving agent selected from benzoic acid, sodium benzoate, sorbic acid or benzyl alcohol.

In one embodiment, a mixture of microalga biomass selected from *Chlorella sorokiniana, Chlorella vulgaris* or *Tetradesmus obliquus* is present in a ratio between 1:2 - 1:10.

In one embodiment, a mixture of at least two polymers is present in a ratio between 1:1 - 1:10.

In one embodiment, the gel formulation further comprises between 0.05% - 0.20% (w/v) of at least one thickening agent selected from ethylene glycol, mannitol or xylitol.

In one embodiment, the microalga biomass is free in the gel formulation.

In one embodiment, the microalga biomass is encapsulated in polymeric particles.

In one embodiment, the polymeric particles are made of at least one polymer selected from alginic acid, carboxymethylcellulose, hyaluronic acid or chitosan.

In one embodiment, the gel formulation comprises between 10 and 30 % (w/w) of particles loaded with microalga biomass.

The invention also relates to a process to obtain the gel formulation for the treatment of drug intoxication comprising the following steps:
- Producing biomass from at least one microalga selected from *Chlorella sorokiniana, Chlorella vulgaris, or Tetradesmus obliquus;*
- Preparing an aqueous solution of at least one polymer selected from alginate, carrageenan, pectin, chitosan, gum arabic, gellan gum, or glucomannan;
- Mixing between 0.5% - 5% (w/v) of the aqueous polymer solution with between 0.5% - 5% (w/v) of microalga biomass of at least one selected from *Chlorella sorokiniana, Chlorella vulgaris, or Tetradesmus obliquus;*
- Adding between 0.05% - 0.10% (w/v) of at least one preserving agent selected from benzoic acid, sodium benzoate, sorbic acid or benzyl alcohol, to the previous mixture;
- Adding between 0.2% - 0.5% (w/v) of a cross-linking agent selected from calcium chloride, magnesium chloride or sodium triphosphate, to the previous mixture;
- Removing air from the mixture to obtain the gel formulation.

In one embodiment, the microalga is added as a paste or freeze-dried.

In one embodiment, when more than one polymer is selected, each polymer is dissolved separately and then added together.

In one embodiment, between 0.05% - 0.20% (w/v) of at least one thickening agent selected from ethylene glycol, mannitol or xylitol, is added to the mixture of at least one polymer and microalga biomass.

In one embodiment, the microalga biomass is encapsulated in polymeric particles made of a polymer selected from alginic acid, carboxymethylcellulose, hyaluronic acid or chitosan.

### General description

The present invention is based on the development of a gel formulation for oral administration incorporated with microalgae biomass (free or encapsulated in particles) capable of adsorbing different drugs in the gastrointestinal tract. The present invention also discloses a process to obtain the formulation.

The integration of microalgae abilities to adsorb drugs with the demand of new efficient methods for the treatment of drugs intoxications constitutes the focus of the present invention. It was possible to validate the use of different microalgal species for the intestinal drug adsorption of paracetamol, ibuprofen, and alprazolam. Furthermore, since the oral administration is envisioned, the microalgae biomass (paste and freeze-dried) was incorporated into particles and then loaded into the formulation aiming to ensure the transport along gastrointestinal tract and protect it against the acidic gastric environment to allow drug adsorption at intestine level.

*Chlorella sorokiniana, Chlorella vulgaris , Tetradesmus obliquus* were selected, considering their drug adsorption efficiencies for ibuprofen, paracetamol and cadmium from wastewater samples, according to the approaches previously developed and reported in previous works [10, 12-15, 27-29].

Then, different concentrations of microalgae biomass and drugs (paracetamol, ibuprofen and alprazolam) were combined at different ratios, and microalgae biomass ability to adsorb the free drugs was evaluated. These assays were crucial to the determine the maximum efficiency of the drug absorption by microalgae biomass.

Then, the microalgae biomass was incorporated into particles and assessed under different physiological pH (Simulated gastric fluid (SGF) and simulated intestinal fluid (SIF)).

The gel formulation aims to provide a safe carrier for the particles loaded with biomass along gastrointestinal tract, presenting a pH-responsive character resistant to acidic gastric environment.

The results obtained with this invention are of paramount importance for the valorisation of microalgae biotechnology as well as for the pharmaceutical industry. Considering the results obtained, the proof-of-concept was further confirmed and validated with complementary assays to assure the translation potential to the industry and clinics.

The present invention can also contribute to reduce the costs associated to the current clinical treatments for drug intoxications by providing a new alternative based on the microalgae biotechnology as well as reducing the use of synthetic chemical compounds. Such strategy is aligned with the recent trend on search and application of natural-based products in biomedical fields. Regarding the economic impact, it is expected that the microalgal market will grow by 2026, with a profit exceeding US$75 million. Moreover, the commercialization of high-value pharmaceutical products from microalgae will likely become a gateway to a multibillion-dollar industry in the near future.

This formulation will be able to withstand the different pH levels characteristic of the physiological environment of the mammalian gastrointestinal tract, due to its qualitative composition based on natural pH-responsive polymers and classified as GRAS. These polymers are biodegradable and are naturally metabolized and excreted by the bodies of living beings.

Thus, the gel formulation will facilitate oral administration, resist gastric pH and, in the intestine, allow the adsorption of drugs by the incorporated microalgae, preventing and/or minimizing the absorption of drugs through the mucosa and thus avoiding and/or reducing toxic effects in the event of drug poisoning.

Thus, this formulation stands out from those on the market because it has distinctive characteristics, since it will be possible to reduce adverse effects in cases of drug poisoning, avoiding the use of aggressive approaches such as gastric lavage or activated charcoal. On the other hand, the formulation is based on polymers of natural origin recognized as GRAS, which allows it to be applied and used clinically. In addition, it will be possible to use a highly differentiating resource - microalgae - as a bioactive agent with the ability to adsorb drugs and, in turn, prevent their absorption and the adverse effects associated with drug poisoning.

The effectiveness of the formulation is associated with the ability of microalgae biomass, namely the species *Chlorella sorokiniana, Chlorella vulgaris, and Tetradesmus obliquus,* to adsorb drugs, their metabolites and heavy metals. The formulation, which contains different polymers and microalgae biomass in different ratios (quantitative and qualitative), guarantees the adsorption of a single drug or combination of drugs in toxic concentrations, thus allowing them to be eliminated from the body.

In addition, the formulation can be available in different volumes depending on its application, i.e. in a clinical environment and to be used by qualified health professionals, the formulation can be stored, for example, in containers with volumes of 500 ml-750 ml; and in cases of emergency kits, the formulation can be prepared, for example, in containers with volumes of 50 ml-150 ml.

Therefore, to obtain the present invention the following was proposed:
i) optimize the microalgal biomass:drug ratios to obtain an efficient drug adsorption efficiency; *Chlorella sorokiniana, Chlorella vulgaris,* and *Tetradesmus obliquus* biomass was used;
ii) immobilize microalgal biomass into particles to confer protection against external harmful effects;
iii) incorporate the particles loaded with microalgae into a gel formulation to facilitate the transport along gastrointestinal tract.

Paracetamol (PCT), ibuprofen (IBU) and alprazolam (APZ) were selected considering epidemiological data for drug intoxications, since they are the cause of the majority of intoxications cases in Portugal.

### Detailed description of embodiments

Now, preferred embodiments of the present application will be described in detail with reference to the annexed drawings. However, they are not intended to limit the scope of this application.

The present invention relates to a gel formulation for the treatment of drug intoxication.

The gel formulation comprises:
- Between 0.5 - 5% (w/v) of microalga biomass selected from at least one of *Chlorella sorokiniana, Chlorella vulgaris* or *Tetradesmus obliquus;*
- Between 0.5% - 5% (w/v) of at least one polymer selected from alginate, carrageenan, pectin, chitosan, gum arabic, gellan gum, or glucomannan;
- Between 0.05% - 0.10% (w/v) of at least one preserving agent selected from benzoic acid, sodium benzoate, sorbic acid or benzyl alcohol.

In one embodiment, a mixture of microalga biomass selected from *Chlorella sorokiniana, Chlorella vulgaris* or *Tetradesmus obliquus* is present in a ratio between 1:2 - 1:10.

In one embodiment, a mixture of at least two polymers is present in a ratio between 1:1 - 1:10.

In one embodiment, the microalga biomass is free in the gel formulation.

In one embodiment, the microalga biomass is in the form of a paste or freeze-dried.

In one embodiment, the gel formulation comprises between 0.05% - 0.20% (w/v) of at least one thickening agent selected from ethylene glycol, mannitol or xylitol.

In another embodiment, the microalga biomass is encapsulated in polymeric particles. In one embodiment, the polymeric particles are made of at least one polymer selected from alginic acid, carboxymethylcellulose, hyaluronic acid or chitosan. In one embodiment the particles have an average diameter size between 300-3000 µm.

In one embodiment, the gel formulation comprises between 10 and 30% (w/w) of particles loaded with microalga biomass.

The present invention also relates to process to obtain the gel formulation for the treatment of drug intoxication comprising the following steps:
- Producing biomass from at least one microalga selected from *Chlorella sorokiniana, Chlorella vulgaris, or Tetradesmus obliquus;*
- Preparing an aqueous solution of at least one polymer selected from alginate, carrageenan, pectin, chitosan, gum arabic, gellan gum, or glucomannan;
- Mixing between 0.5% - 5% (w/v) of the aqueous polymer solution with between 0.5% - 5% (w/v) of microalga biomass of at least one selected from *Chlorella sorokiniana, Chlorella vulgaris, or Tetradesmus obliquus*;
- Adding between 0.05% - 0.10% (w/v) of at least one preserving agent selected from benzoic acid, sodium benzoate, sorbic acid or benzyl alcohol, to the previous mixture;
- Adding between 0.2% - 0.5% (w/v) of a cross-linking agent selected from calcium chloride, magnesium chloride or sodium triphosphate, to the previous mixture;
- Removing air from the mixture to obtain the gel formulation.

In one embodiment, the culture conditions to produce *Chlorella sorokiniana* biomass are in an autotrophic (with the presence of light), heterotrophic (without the presence of light in fermenters) or mixotrophic (combination of the two previous modes) mode. They are easily grown in medium containing inorganic nutrients (nitrogen, phosphorus and CO₂) which are required by microalgae at approximately 180% of their dry weight. Different options and concentrations of culture medium can be used, as described in the literature (e.g. BG-11 and BBM). Similarly, optimal conditions are required for temperature, pH of 6.5-7.0, and light intensity, once directly influences the growth and development of microalgal biomass. For *Chlorella sorokiniana* best performance in growth and biomass productivity were achieved for culture conditions of 20-35 °C, 3000-7000 Lux, and in heterotrophy, with glucose, or Acetic acid in mixotrophy under continuous illumination and agitation (100 to 1200 rpm) [45, 46, 47].

In one embodiment, the culture conditions to produce *Chlorella vulgaris* biomass are in an autotrophic (with the presence of light), heterotrophic (without the presence of light in fermenters) or mixotrophic (combination of the two previous modes) mode. They are easily grown in medium containing inorganic nutrients (nitrogen, phosphorus and CO₂) which are required by microalgae at approximately 180% of their dry weight. Different options and concentrations of culture medium can be used, as described in the literature (e.g. BG-11 and BBM). Similarly, optimal conditions are required for temperature, pH and light intensity, once directly influence the growth and development of microalgal biomass. For *Chlorella vulgaris* best performance in growth and biomass productivity were achieved for culture conditions of 20-35 °C, 3000-7000 Lux, and in heterotrophic and mixotrophic cultivation with glucose, under agitation (100 to 1200 rpm) [46, 47]

In one embodiment, the culture conditions to produce *Tetradesmus obliquus* biomass are in an autotrophic (with the presence of light), heterotrophic (without the presence of light in fermenters) or mixotrophic combination of the two previous modes) mode. They are easily grown in medium containing inorganic nutrients (nitrogen, phosphorus and CO₂) which are required by microalgae at approximately 180% of their dry weight. Different options and concentrations of culture medium can be used, as described in the literature (e.g. BG-11, BBM). Similarly, optimal conditions are required for temperature, pH and light intensity, once directly influence the growth and development of microalgal biomass. For *Tetradesmus obliquus* best performance in growth and biomass productivity were achieved for culture conditions of 20-25°C with an illumination of 2000-7000 lux, at pH of 6.5-7.5 and in heterotrophy, with glucose, or supplementing culture medium, in mixotrophy under continuous illumination and agitation (100 to 1000 rpm) [48].

In one embodiment, the microalga is added as a paste or freeze-dried.

In one embodiment, when more than one polymer is selected, each polymer is dissolved separately and then added together.

In one embodiment, an aqueous solution of pectin is obtained by dissolution with sodium citrate.

In one embodiment, between 0.05% - 0.20% (w/v) of at least one thickening agent selected from ethylene glycol, mannitol or xylitol, is added to the mixture of at least one polymer and microalga biomass.

In one embodiment, the air is removed from the mixture by sonication and/or vacuum.

In another embodiment, the microalga biomass is encapsulated in polymeric particles. In one embodiment, the polymeric particles are made of at least one polymer selected from alginic acid, carboxymethylcellulose, hyaluronic acid or chitosan. In one embodiment the particles have an average diameter size between 300-3000 µm.

In one embodiment, the encapsulation of microalga biomass is carried out with known precipitation methods, promoting the ionic and/or chemical cross-linking. The microalga, or mixtures thereof, are mixed with at least one polymer. The mixture is then added to a solution of cross-linker as drops to obtain the particles loaded with microalgae.

In one embodiment, the cross-linker used to obtain the particles is selected from calcium chloride, magnesium chloride, sodium tripolyphosphate, 1,4-butanediol diglycidyl ether, among others.

The presently disclosed gel formulation is suitable for the treatment of drug intoxication in humans or animals, therefore finding application in pharmaceutical, medical and veterinary technical fields.

In one embodiment, the ratio between particles (1g) and drug that can be adsorbed for the treatment of drug intoxication is between 30-60 mg of IBU and 5-18 mg of PCT.

To obtain the gel composition disclosed herein, the following considerations were taken into account:

### 1. Characterization the optimal conditions and ratios of microalgae biomass for drug adsorption

The selected microalgae were cultivated, where the culture conditions (culture medium, pH, light) were optimized for biomass production, in batch and/or semicontinuous cultures according to the approach previously validated for different species [10,27,28]. Then, the microalgae biomass was harvested, centrifuged, and freeze-dried, following the conditions described in literature [31,32].

The characterization of biomass was achieved by Fourier-transform infrared spectroscopy (FTIR) and UV-visible spectroscopy (UV-Vis) analysis for identification of main chemical functional groups [33-35].

### 2. Production and characterisation of particles

Natural polymers classified as GRAS and pH-responsiveness (e.g. alginic acid, carboxymethyl cellulose, hyaluronic acid or chitosan) were considered for the particles production by precipitation technique [30]. Settings such as polymer concentration, crosslinker concentration, flow rate, stirring velocity and gelation time were optimized to obtain particles with suitable morphological properties (300-3000 pm) [41]. particles morphology and surface was characterized by Scanning Electron Microscopy (SEM).

Then, the swelling and degradation profile was also assessed under different physiological pH conditions by using simulated intestinal fluid and simulated gastric fluid.

The particles were produced by dissolving the microalgae biomass in the polymeric solution before the ionic crosslinking. Then, the particles morphological and physicochemical properties were evaluated. After that, batch drug adsorption experiments were performed for the particles incorporated into gel formulation. The drug adsorption performance was assessed by UV-VIS and HPLC. Further, the stability of the particles was evaluated.

### 3. Incorporation of the particles into a gel formulation

A gel formulation was produced by using GRAS and pH-responsive natural polymers (e.g. alginate, carrageenan, pectin, chitosan, Arabic gum, gellan gum, and glucomannan) to act as a carrier for the particles and protecting from the acidic gastric pH. All the materials used in the formulation were sterilized and manipulated under aseptic conditions.

The incorporation and distribution of particles in the gel formulation was evaluated by SEM and Confocal Light Scattering Microscopy (CLSM). The gel formulation integrity and resistance to gastric pH was evaluated, and further, the stability, storage conditions, viscosity and pH measurements were performed according to EMA guidelines [43].

### 4. Adsorption experiments

Batch adsorption experiments on the adsorptive removal of selected drugs by microalgae biomass were carried out adapting the methodology validated for freeze dried biomass of Scenedesmus obliquus by Silva et al. [9]. So, the freeze-dried microalgae biomass (different concentrations) was incubated with drugs (different concentrations), and at different ratios, at 37 °C under stirring, in the SIF and SGF (fluids that mimic the environments of the gastrointestinal tract, prepared following the protocol described by European Pharmacopeia 10.0) [36,37]. Then, the microalgae biomass was characterized by FTIR, UV-Vis. Drug adsorption efficiency was determined by UV-Vis and/or High-Performance Liquid Chromatography (HPLC) [38] for adsorption kinetics assessment [9].

### Examples:

### 1) Gel formulation composition

A gel formulation was prepared with a mixture of GRAS and pH-responsive natural polymers, alginate and pectin, (table 1) to act as a carrier for the particles with microalgae biomass and protecting them from the acidic gastric pH. The preparation of the gel involved the dissolution of pectin (1-2% w/v) in sodium citrate (0.10-0.4% w/v) under stirring, followed by the addition of sodium alginate (0.25-1% w/v). After the complete dissolution calcium chloride was added (0.008-0.064% (w/v)).

**Table 1. Gel composition**

| **Sodium Alginate (% w/v)** | **Pectin (% w/v)** | **Calcium Chloride (% w/v)** | **Sodium Citrate (% w/v)** |
|---|---|---|---|
| 0.25 - 1 | 1 -2 | 0.008 - 0.064 | 0.10- 0.4 |

The gel formulation integrity and resistance to gastric pH was evaluated, and further, the stability, storage temperature, porosity, viscosity and pH monitored according to EMA guidelines and European Pharmacopeia 10.0 (5.17.1.) by using simulated gastric fluid (SGF) and simulated intestinal fluid (SIF) composed by sodium chloride and hydrochloric acid at pH 1.6 and sodium hydroxide, monobasic potassium phosphate and pancreatin at pH 6.8, respectively. The gelation of the formulation occurred under acidic conditions (SGF) as a result of intermolecular junction zones formed by the union of the Ca²⁺ ions with guluronic acid residues of alginate or galacturonic acid residues in pectin of the adjacent polymer chain, producing an "egg-box" configuration. Otherwise, under SIF conditions, the low concentration of H⁺ does not support the calcium chloride ionization and the gelation process is reverted.

The incubation of the gel in acidic pH (SGF) resulted in a minimal weight loss of 4% during the 120 min, such is justified by the formation of intermolecular junctions that stabilize the gel. In turn, when incubated in SIF, the alkalinization of the pH promoted the electrostatic repulsion between the sodium alginate and pectin chains. Therefore, the gel loses its structure and degrades, reaching 87% after the 30 min of incubation in SIF. This will support the release of particles that can act as adsorption agents. In turn, the stability of the gel formulation was confirmed by measuring the pH variations for 28 days, under room temperature and refrigerated storage conditions. The results revealed that the variation was just 0.14, remaining the pH value stable around 5.7 and 6.11, demonstrating potential for oral administration.

The gel showed a medium viscosity of 14.72 mPa and a maximum sheer force of 1125 (¹/s). These values indicate that the gel has a non-Newtonian behaviour since the viscosity decreases with the increase in the shear forces. Therefore, the produced gel is compatible with the oral administration of the gel and its removal from a conventional package. Moreover, low viscosity values are associated with easier administration and swallowing.

During the incubation of the gel in SGF fluid it is observed a reduction in the porosity from 30% to 10% after 30 minutes, remaining this value constant until 60 minutes. Such value further supports the gelation behaviour of the gels upon contact with SGF.

The analysis of the swelling profile shows that the peak of the gel's swelling occurs at the 60 minutes, reaching the 52.9%, which stabilized until the 120 minutes.

### 2) particles production

After the production of the gel formulation, particles with microalgae biomass were incorporated therein. For the production of the particles, the biomass of each microalgae mentioned above was individually dissolved in the polymeric solution (according to Tables 2.1 and 2.2) before ionic crosslinking with calcium chloride.

**Table 2.1. particles production**

| **Formulation** | **Sodium Alginate (% w/v)** | **Microalgae (% w/v)** | **Chitosan (% w/v)** | **Calcium Chloride (% w/v)** |
|---|---|---|---|---|
| A | 2 | 0.64 | -- | 2 |
| B to F | 2 | 0.64 | 0.5 | 0.09 |

**Table 2.2. Production of gel loaded with chitosan particles (MPs) comprising biomass of each microalgae mentioned above**

| | | | | | |
|---|---|---|---|---|---|
| **Formulation** | **Sodium Alginate (% w/v)** | **Pectin (% w/v)** | **Calcium Chloride (% w/v)** | **Sodium Citrate (% w/v)** | **MPs** |
| | 0.5 | 1 | 0.016 | 0.17 | 1g per 4g of gel |

### 3) Biosorption studies

Batch drug adsorption experiments were performed under physiological pH characteristic for gastric (SGF) and intestine environment (SIF), accordingly to the European Pharmacopeia. The drug adsorption performance of the gel formulations was assessed by UV-VIS and HPLC.

Experiments were performed at an initial concentration of IBU and PCT (1 g/L each), pH (1.24- 6.69, corresponding to SGF and SIF)) and gel formulation (5 g, including 1g of particles) in 100 mL of SGF and SIF stirred at 150 rpm in a shaking incubator for 4 h (2h in SGF and 2h in SIF). The temperature was maintained at 37 _{°}C throughout the experiments.

After biosorption the IBU and PCT concentration in the supernatant (corresponding to the non-adsorbed) was determined using HPLC accordingly to European Pharmacopeia. The maximum biosorption capacity and removal efficiency of IBU and PCT was calculated from Tables 3.1.

**Table 3.1 Biosorption capacity and removal efficacy of IBU and PCT (1g/L each) from SGF and SIF with the formulation of Table 2.2**

| | *C*. *vulgaris* paste | *C*. *vulgaris* freeze-dried | *Tetradesmus obliquus* paste | *Tetradesmus obliquus* freeze-dried |
|---|---|---|---|---|
| IBU | 43 mg | 53 mg | 31 mg | - - |
| PCT | 5 mg | 6 mg | 18 mg | 5 mg |

### 4) Molecular Dynamics Simulations for the interaction of drugs with lipid bilayers of microalgae

Herein is reported the study of the interaction of several drugs with lipid bilayers, using molecular dynamics simulations. The selected drugs were alprazolam, paracetamol, ibuprofen, warfarin, bromethalin and brodifacoum. This set of drugs was selected based on their prevalence on drug intoxication reports both for humans and pets. Here, alprazolam, paracetamol and ibuprofen are of higher relevance for human drug intoxication, being warfarin, bromethalin and brodifacoum more relevant for animals' intoxication. The drug/membrane interaction was characterized regarding the visualization and analysis of the preferential location of the drugs while inserted in the lipid bilayers. Furthermore, the affinity of the drugs for lipid membrane models was addressed by calculating the free energy profiles for their interaction. This characterization reveals insights about the requirements for the development of innovative solutions to fight drug intoxication, such as the optimization of microalgae-based nanoparticles and liposomes used by oral administration.

Two lipid membrane models were used in this study, one composed only by 1-palmitoyl, 2-oleoyl-sn-glycero-3-phosphocholine (POPC), another developed to mimic the lipid membrane composition of *Chlorella vulgaris.* The pure membrane systems were built using the Membrane Builder tool from CHARMM-GUI software.

Lipid bilayers composed by POPC were used as this lipid is the most abundant lipid in cell membranes, and therefore conventionally used in this type of simulations. For this membrane system, a total of 256 lipids and 50 water molecules/lipid were added. The composition of the Chlorella membrane model was based on experimental data reported in the literature.

The construction of the systems containing the drugs was done placing four drug molecules positioned at different regions of the lipid membrane.

The observed behaviour of interaction is specific for each drug; however, some general conclusions could be obtained. More hydrophobic molecules tend to be deeply inserted in the membrane environment, also having the possibility to fast equilibrate between lipid membrane leaflets. Regarding the strength of the interaction, the analysis of the obtained free energy profiles lead to conclude that the tested drugs have preference for the interaction with the lipid membrane environment, being able to compete with drug absorption process and work as a solution in cases of drug intoxication.

This characterization is important for the development of innovative solutions to solve problems related to drug intoxication, both in humans and in pets, such as the gel formulation disclosed in the present patent application.

### Blibliography

1. De Luca M,Pappalardo I,Limongi AR,Viviano E, Radice RP,Todisco S,Martelli G,Infantino V,Vassallo A. Lipids from Microalgae for Cosmetic Applications. Cosmetics 2021;8 (2) : 52.
2.Prosenc F,Piechocka J,Škufca D,Heath E,Griessler Bulc T,Istenič D,Buttiglieri G. Microalgae-based removal of contaminants of emerging concern:Mechanisms in Chlorella vulgaris and mixed algal-bacterial cultures. Journal of Hazardous Materials. 2021/09/15/ 2021;418:126284.
3. Miguel SP,Ribeiro MP,Otero A,Coutinho P. Application of microalgae and microalgal bioactive compounds in skin regeneration. Algal Research. 2021/10/01/ 2021;58:102395.
4.Hu H,Zhong D,Li W,Lin X,He J, Sun Y, Wu Y, Shi M, Chen X, Xu F, Zhou M. Microalgae-based bioactive hydrogel loaded with quorum sensing inhibitor promotes infected wound healing. Nano Today. 2022/02/01/ 2022;42:101368.
5.Ismail D, Prakasa M, Nugroho P. The effect of adding microalgae extract Spirulina platensis containing flavonoid in the formation of Sunscreen towards cream stability and SPF values. Vol 22552020.
6.Garcia JL, de Vicente M, Galán B. Microalgae, old sustainable food and fashion nutraceuticals. Microbial biotechnology. 2017;10(5):1017-1024.
7.Saavedra R, Muñoz R, Taboada ME, Vega M, Bolado S. Comparative uptake study of arsenic, boron, copper, manganese and zinc from water by different green microalgae. Bioresource Technology. 2018/09/01/ 2018;263:49-57.
8.Escapa C, Coimbra RN, Nuevo C, Vega S, Paniagua S, Garcia AI, Calvo LF, Otero M. Valorization of Microalgae Biomass by Its Use for the Removal of Paracetamol from Contaminated Water. Water. 2017;9(5):312.
9.Silva A, Coimbra RN, Escapa C, Figueiredo SA, Freitas OM, Otero M. Green Microalgae Scenedesmus Obliquus Utilization for the Adsorptive Removal of Nonsteroidal Anti-Inflammatory Drugs (NSAIDs) from Water Samples. International Journal of Environmental Research and Public Health. 2020;17(10):3707.
10. Coutinho P; Alves, A; Martins, I; Ribeiro, MP; Rodrigues, M; Araujo, ARTS. PW199 Effects of 17-b-estradiol and diclofenac on growth and chlorophyllcontent of Chlorella vulgaris. 8th Congress of European Microbiologists - FEMS 2019; 7-11 July 2019, 2019; Glasgow, Scotland.
11. Sousa H, Sousa CA, Simões LC, Simões M. Microalgal-based removal of contaminants of emerging concern. Journal of Hazardous Materials. 2022/02/05/ 2022;423:127153.
12. Encarnação T, Aguiar A, Palito C, Pais AACC, Campos MG, Sobral AJFN, Burrows HD. Development and validation of a RP-HPLC method for the simultaneous analysis of paracetamol, ibuprofen, olanzapine, and simvastatin during microalgae bioremediation. MethodsX. 2020/01/01/ 2020;7:101083.
13. Escapa C, Coimbra RN, Paniagua S, Garcia AI, Otero M. Comparison of the culture and harvesting of Chlorella vulgaris and Tetradesmus obliquus for the removal of pharmaceuticals from water. Journal of Applied Phycology. 2017/06/01 2017;29(3):1179-1193.
14. Escapa C, Coimbra RN, Paniagua S, Garcia AI, Otero M. Paracetamol and salicylic acid removal from contaminated water by microalgae. Journal of Environmental Management. 2017/12/01/ 2017;203:799-806.
15. Xiong Q, Hu L-X, Liu Y-S, Zhao J-L, He L-Y, Ying G-G. Microalgae-based technology for antibiotics removal: From mechanisms to application ofinnovational hybrid systems. Environment International. 2021/10/01/ 2021;155:106594.
16. Santaeufemia S, Torres E, Abalde J. Biosorption of ibuprofen from aqueous solution using living and dead biomass of the microalga Phaeodactylumtricornutum. Journal of Applied Phycology. 2018/02/01 2018;30(1):471-482.
17. INEM. Relatório de Atividades específicas desenvolvidas nos CODU 2020. 2020.
18.INEM. Indicadores de Desempenho do INEM. 2016; https://extranet.inem.pt/stats/?stat=30&stats=31&ano=2016. Accessed February 18, 2022.
19.Mégarbane B, Oberlin M, Alvarez J-C, Balen F, Beaune S, Bédry R, Chauvin A, Claudet I, Danel V, Debaty G, Delahaye A, Deye N, Gaulier J-M,
   Grossenbacher F, Hantson P, Jacobs F, Jaffal K, Labadie M, Labat L, Langrand J, Lapostolle F, Le Conte P, Maignan M, Nisse P, Sauder P, Tournoud C,
   Vodovar D, Voicu S, Claret P-G, Cerf C. Management of pharmaceutical and recreational drug poisoning. Annals of Intensive Care. 2020/11/23 2020;10(1):157.
20. Alwan IA, Awadh AI, Tangiisuran B, Khan HRM, Yahaya N, Majid MI. Pharmaceuticals Poisoning: Reported by the National Poison Centre in Malaysiabetween 2010 and 2015. J Pharm Bioallied Sci. Oct-Dec 2020;12(4):475-481.
21. Chyka PA, Seger D, Krenzelok EP, Vale JA. Position paper: Single-dose activated charcoal. Clin Toxicol (Phila). 2005;43(2):61-87.
22.Vale JA, Kulig K. Position paper: gastric lavage. J Toxicol Clin Toxicol. 2004;42(7):933-943.
23.Martins SS, Sampson L, Cerdá M, Galea S. Worldwide Prevalence and Trends in Unintentional Drug Overdose: A Systematic Review of the Literature. American Journal of Public Health. 2015;105(11) :e29-e49.
24. Vieira MV, Pastrana LM, Fuciños P. Microalgae Encapsulation Systems for Food, Pharmaceutical and Cosmetics Applications. Marine Drugs. 2020;18(12):644.
25. Shah SA, Sohail M, Minhas MU, Nisar ur R, Khan S, Hussain Z, Mudassir, Mahmood A, Kousar M, Mahmood A. pH-responsive CAP-co-poly(methacrylicacid)-based hydrogel as an efficient platform for controlled gastrointestinal delivery: fabrication, characterization, in vitro and in vivo toxicity evaluation. Drug Delivery and Translational Research. 2019/04/01 2019;9(2):555-577.
26. Yin Z-C, Wang Y-L, Wang K. A pH-responsive composite hydrogel beads based on agar and alginate for oral drug delivery. Journal of Drug Delivery Science and Technology. 2018/02/01/ 2018;43:12-18.
27. Coutinho P, Ferreira M, Freire I, Otero A. Enriching Rotifers with "Premium" Microalgae: Rhodomonas lens. Marine Biotechnology. 2020/02/01 2020;22(1) :118-129.
28. Dominguez A, Ferreira M, Coutinho P, Fabregas J, Otero A. Delivery of astaxanthin from Haematococcus pluvialis to the food chain. Aquaculture. 01/01 2005;250:424-430.
29. Ferreira M, Coutinho P, Seixas P, Fábregas J, Otero A. Enriching Rotifers with "Premium" Microalgae. Nannochloropsis gaditana. Marine Biotechnology. 2009/10/01 2009;11 (5) :585-595.
30. Ribeiro MP, Morgado PI, Miguel SP, Coutinho P, Correia IJ. Dextran-based hydrogel containing chitosan microparticles loaded with growth factors to be used in wound healing. Mater Sci Eng C Mater Biol Appl. Jul 1 2013;33 (5) :2958-2966.
31. Santaeufemia S, Torres E, Mera R, Abalde J. Bioremediation of oxytetracycline in seawater by living and dead biomass of the microalga Phaeodactylum tricornutum. Journal of Hazardous Materials. 2016/12/15/ 2016;320:315-325.
32.Habibzadeh M, Chaibakhsh N, Naeemi AS. Optimized treatment of wastewater containing cytotoxic drugs by living and dead biomass of the freshwater microalga, Chlorella vulgaris. Ecological Engineering. 2018/02/01/ 2018;111:85-93.
33. Miguel SP, Loureiro J, Ribeiro MP, Coutinho P. Osmundea sp. macroalgal polysaccharide-based nanoparticles produced by flash nanocomplexation technique. Int J Biol Macromol. Feb 2 2022;204:9-18.
34.Jacinto TA, Rodrigues CF, Moreira AF, Miguel SP, Costa EC, Ferreira P, Correia IJ. Hyaluronic acid and vitamin E polyethylene glycol succinate functionalized gold-core silica shell nanorods for cancer targeted photothermal therapy. Colloids and Surfaces B: Biointerfaces. 2020/04/01/ 2020;188:110778.
35.Sequeira RS, Miguel SP, Cabral CSD, Moreira AF, Ferreira P, Correia IJ. Development of a poly(vinyl alcohol)/lysine electrospun membrane-based drug delivery system for improved skin regeneration. Int J Pharm. Oct 30 2019;570:118640.
36.Almeida A, Araújo M, Novoa-Carballal R, Andrade F, Gonçalves H, Reis RL, Lúcio M, Schwartz S, Sarmento B. Novel amphiphilic chitosan micelles as carriers for hydrophobic anticancer drugs. Materials Science and Engineering: C. 2020/07/01/ 2020;112:110920.
37.Saúd I-Md. 9, Farmacopeia Portuguesa 9. Lisboa; 2008.
38. Rodrigues DA, Miguel SP, Loureiro J, Ribeiro M, Roque F, Coutinho P. Oromucosal Alginate Films with Zein Nanoparticles as a Novel Delivery System for Digoxin. Pharmaceutics. 2021;13(12):2030.
39. Mukhopadhyay P, Chakraborty S, Bhattacharya S, Mishra R, Kundu PP. pH-sensitive chitosan/alginate core-shell nanoparticles for efficient and safeoral insulin delivery. Int J Biol Macromol. 2015/01/01/ 2015;72:640-648.
40. Castro CM, Pinheiro M, Lúcio M, Giner-Casares JJ, Camacho L, Lima JLFC, Reis S, Segundo MA. Insights about α-tocopherol and Trolox interaction with phosphatidylcholine monolayers under peroxidation conditions through Brewster angle microscopy. Colloids and Surfaces B: Biointerfaces. 2013/11/01/ 2013;111:626-635.
41. Bertoni S, Albertini B, Facchini C, Prata C, Passerini N. Glutathione-Loaded Solid Lipid Microparticles as Innovative Delivery System for OralAntioxidant Therapy. Pharmaceutics. 2019;11(8):364.
42. Miguel SP, Ribeiro MP, Brancal H, Coutinho P, Correia IJ. Thermoresponsive chitosan-agarose hydrogel for skin regeneration. Carbohydrate Polymers. 2014/10/13/ 2014;111:366-373.
43. Agency EM. ICH Topic Q 1 A (R2) Stability Testing of new Drug Substances and Products. In: Agency EM, ed2003.
44. https://www.inem.pt/wp-content/uploads/2022/03/CIAV-DADOS-ESTATISTICOS-2021.pdf
45. Taghavijeloudar, M., Yaqoubnejad, P., Amini-Rad, H. et al. Optimization of cultivation condition of newly isolated strain Chlorella sorokiniana pa.91 for CO2 bio-fixation and nutrients removal from wastewater: Impact of temperature and light intensity. Clean Techn Environ Policy 25, 589-601 (2023). https://doi.org/10.1007/s10098-021-02199-5
46. Wilson G. Morais Junior, Malihe Gorgich, Priscila S. Corrêa, António A. Martins, Teresa M. Mata, Nídia S. Caetano, Microalgae for biotechnological applications: Cultivation, harvesting and biomass processing, Aquaculture, Volume 528, 2020, 735562
47. Aswathy Udayan, Ranjna Sirohi, Nidhin Sreekumar, Byoung-In Sang, Sang Jun Sim, Mass cultivation and harvesting of microalgal biomass: Current trends and future perspectives, Bioresource Technology, Volume 344, Part B, 2022, 126406
48. Jihed Bentahar, Jean-Sébastien Deschênes, Media optimization design towards maximizing biomass production of Tetradesmus obliquus under mixotrophic conditions, Bioresource Technology Reports, Volume 17, 2022, 100885.

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

## Claims

1. A gel formulation for the treatment of drug intoxication comprising:
- Between 0.5 - 5% (w/v) of microalga biomass selected from at least one of *Chlorella sorokiniana, Chlorella vulgaris* or *Tetradesmus obliquus*;
- Between 0.5% - 5% (w/v) of at least one polymer selected from alginate, carrageenan, pectin, chitosan, gum arabic, gellan gum, or glucomannan;
- Between 0.05% - 0.10% (w/v) of at least one preserving agent selected from benzoic acid, sodium benzoate, sorbic acid or benzyl alcohol.

2. The gel formulation according to the previous claim, wherein a mixture of microalga biomass selected from *Chlorella sorokiniana, Chlorella vulgaris* or *Tetradesmus obliquus* is present in a ratio between 1:2 - 1:10.

3. The gel formulation according to any of the previous claims, wherein a mixture of at least two polymers is present in a ratio between 1:1 - 1:10.

4. The gel formulation according to any of the previous claims, wherein the gel formulation further comprises between 0.05% - 0.20% (w/v) of at least one thickening agent selected from ethylene glycol, mannitol or xylitol.

5. The gel formulation according to any of the previous claims, wherein the microalga biomass is free in the gel formulation.

6. The gel formulation according to any of the claims 1 to 4, wherein the microalga biomass is encapsulated in polymeric particles.

7. The gel formulation according to the previous claim, wherein the polymeric particles are made of a polymer selected from alginic acid, carboxymethylcellulose, hyaluronic acid or chitosan.

8. The gel formulation according to any of the claims 6 to 7, wherein the gel formulation comprises between 10 and 30 % (w/w) of particles loaded with microalga biomass.

9. A process to obtain the gel formulation described in any of the previous claims for the treatment of drug intoxication comprising the following steps:
- Producing biomass from at least one microalga selected from *Chlorella sorokiniana, Chlorella vulgaris, or Tetradesmus obliquus;*
- Preparing an aqueous solution of at least one polymer selected from alginate, carrageenan, pectin, chitosan, gum arabic, gellan gum, or glucomannan;
- Mixing between 0.5% - 5% (w/v) of the aqueous polymer solution with between 0.5% - 5% (w/v) of microalga biomass of at least one selected from *Chlorella sorokiniana, Chlorella vulgaris, or Tetradesmus obliquus;*
- Adding between 0.05% - 0.10% (w/v) of at least one preserving agent selected from benzoic acid, sodium benzoate, sorbic acid or benzyl alcohol, to the previous mixture;
- Adding between 0.2% - 0.5% (w/v) of a cross-linking agent selected from calcium chloride, magnesium chloride or sodium triphosphate, to the previous mixture;
- Removing air from the mixture to obtain the gel formulation.

10. The process according to the previous claim, wherein the microalga is added as a paste or freeze-dried.

11. The process according to any of the claims 9 to 10, wherein when more than one polymer is selected, each polymer is dissolved separately and then added together.

12. The process according to any of the claims 9 to 11, wherein between 0.05% - 0.20% (w/v) of at least one thickening agent selected from ethylene glycol, mannitol or xylitol, is added to the mixture of at least one polymer and microalga biomass.

13. The process according to any of the claims 9 to 12, wherein the microalga biomass is encapsulated in polymeric particles made of at least one polymer selected from alginic acid, carboxymethylcellulose, hyaluronic acid or chitosan.
